# EUROPEAN PATENT APPLICATION

(11) **EP 1 857 114 A1**
(43) Date of publication of application: **21.11.2007**
(21) Application number: 06728775.5
(22) Date of filing: 08.03.2006
(51) Int. Cl.: A61K 38/00, A61K 9/72, A61K 47/24, A61K 47/26, A61K 47/38, A61P 29/00

(54) **PARTICLE AND PREPARATION CONTAINING THE PARTICLE**

(30) Priority: 09.03.2005 JP 2005066158
(71) Applicant: ONO PHARMACEUTICAL CO., LTD., Chuo-ku Osaka 541 (JP)
(72) Inventor: MASUDA, Hideo, home, Shimamoto-cho, Mishima-gun, Osaka, (JP); SUGIHARA, Hikaru, home, Shimamoto-cho, Mishima-gun, Osaka, (JP); NISHIURA, Akio, home, Shimamoto-cho, Mishima-gun, Osaka, (JP); HAYASHI, Kazuyuki, home, Shimamoto-cho, Mishima-gun, Osaka, (JP)
(74) Representative: Henkel, Feiler & Hänzel
(86) International application number: PCT/JP2006/304506
(87) International publication number: WO 2006/095788

(57) **Abstract**

The present invention relates to a particle having a mean particle size of 0.01 to 20 µm, containing tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate. A preparation containing the particle is excellent in pulmonary delivery through inhalation and is easy to handle because of excellent dispersibility of the particle, and thus the present compound can be used as a pulmonary preparation.

## Description

### Technical Field

The present invention relates to a particle having a given particle size, comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate.

More particularly, the present invention relates to:
(1) a particle having a mean particle size of 0.01 to 20 µm, comprising tert-butyl (4R)-4-{[((1R)-2-[(1-benzylpiperidin-4-yl)amino]-1-{[(cyclohexylmethyl)thio]methyl}-2-oxaethyl)amino]carbonyl}-1,3-thiazolidine-3-carboxylate,
(2) a method for producing the same, and
(3) a preparation using the particle.

### Background Art

Tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate (hereinafter abbreviated to a compound 1) or a salt thereof is useful as a preventive and/or a therapeutic agent for pain (for example, neuropathic pain, cancerous pain, intractable pain, post-operative pain or the like) because it inhibits an N-type calcium channel (Patent Document 1).

However, since the compound 1 is susceptible to metabolism in the digestive tract or liver and the like, it is impossible to orally administer the compound 1 in an effective dose. Also, the compound 1 is poorly soluble in water and therefore it was difficult to use the compound 1 as an injection.

Thus, the present inventors have studied about nasal administration of this compound. In the nasal administration, a bioavailability could be improved by forming the compound into an amorphous formulation (Patent Document 2). However, since nasal mucosa has many developed nervous tissues, in the nasal administration, there is a transient irritation due to the compound 1 and a limitation in surface area of nasal mucosa, and thus it was difficult to perform nasal administration in a high dose.

Also, the compound 1 had such a problem that it is difficult to be formed into a fine powder because of its adhesion and cohesiveness.

Since a crystal obtained by recrystallization of the compound 1 has a mean particle size of about 120 µm, use of such a particle causes problems such as a low bioavailability in the oral administration, poor solubility in water in the intravenous administration, and higher *in vivo* absorptivity than that in the oral administration, but a limitation in a dose due to an irritant property of the compound 1 and narrow surface area in the nasal administration. Therefore, these administration routes were not sufficient to use the compound as a drug.
[Patent Document 1] International Publication No. 00/00470 pamphlet
[Patent Document 2] International Publication No. 2004/113332 pamphlet

### Disclosure of the Invention

### Problems to be Solved by the Invention

An object of the present invention is to provide a particle which contains the compound 1 and is excellent in *in vivo* absorption and also has good dispersibility, and a preparation comprising the same, which is convenient in handling.

### Means for Solving the Problems

In view of the above problems, the present inventors have intensively studied and found that control of the particle size of a particle comprising the compound 1 within a range from about 0.01 to about 20 µm enables attainment of good dispersibility and, surprisingly, an improvement in absorptivity by pulmonary inhalation administration. Also, the present inventors have found that an excellent preparation comprising the particle of the present invention can be obtained by designing to improve absorptivity in the case of inhaling the compound 1 into the lung.

Namely, the present invention provides:
[1] A particle having a mean particle size of 0.01 to 20 µm, comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate;
[2] The particle according to [1], which has a mean particle size of 0.03 to 5 µm;
[3] The particle according to [1], comprising at least one kind selected from a water-soluble polymer and a phospholipid;
[4] The particle according to [3], wherein the water-soluble polymer is at least one kind selected from hydroxypropylmethyl cellulose, hydroxypropyl cellulose and methyl cellulose and the phospholipid is at least one kind selected from soybean lecithin and hydrogenated soybean lecithin;
[5] The particle according to [3], which is produced by comminution in water;
[6] The particle according to [1], further comprising a sugar;
[7] The particle according to [6], wherein the sugar is lactose, glucose or D-mannitol;
[8] A method for producing a particle having a mean particle size of 0.03 to 5 µm, comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate, comprising the step of comminuting a particle of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in water in the presence of at least one kind selected from a water-soluble polymer and a phospholipid;
[9] A preparation comprising the particle according to any one of [1] to [7] ;
[10] The preparation according to [9], which is a preparation for pulmonary administration;
[11] The preparation according to [9], which is produced by comminution in water and further granulation or freeze-drying;
[12] A method for producing a preparation comprising a particle having a mean particle size of 0.03 to 5 µm and comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate, which comprises the steps of comminuting a particle of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in the presence of at least one selected from a water-soluble polymer and a phospholipid, and granulating the comminuted particle through spray drying;
[13] A container for inhalation, comprising the preparation according to [10]; and
[14] The particle according to [1], wherein the content of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in the entire particle is from 60 to 100 w/w%.

Hereinafter, the particle comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate (hereinafter referred to as a compound 1) of the present invention may be sometimes abbreviated to the particle of the present invention.

### Effect of the Invention

A preparation for pulmonary administration using the particle of the present invention exhibits high *in vivo* delivery, namely, a pulmonary delivery rate of about 10 to 100%, and therefore it becomes possible to exhibit sufficient efficacy even in a low dose. Also, it becomes possible to use the compound 1 in a low dose in the particle of the present invention, and thus it also becomes possible to reduce a patient's burden because of reduction of the side effect due to reduction in the dose.

### Best Mode for Carrying Out the Invention

The compound 1 as a main ingredient of the particle of the present invention is described in Patent Document 1 described above and can be produced by a known method.

In the present invention, a "particle" containing the compound 1 may be a particle composed only of the compound 1, or may contain the compound 1 and further may contain at least one kind selected from a water-soluble polymer and a phospholipid, and also may further contain at least one kind of sugars and may be formed into a preparation by adding another preparation base (for example, sugar and the like).

The "mean particle size" means a particle size which represents a group of particles, when the group of particles is composed of a lot of particles each having a non-uniform size. The mean particle size includes, but is not limited to, weighting averages such as a number mean diameter, a length mean diameter, an area mean diameter, a volume mean diameter and the like, and means the volume mean diameter in the present invention.

The mean particle size, namely, the volume mean diameter of the particle of the present invention is preferably from about 0.01 to about 20 µm, more preferably from about 0.03 to about 10 µm, still more preferably from about 0.03 to about 5 µm, and particularly preferably from about 0.1 to about 3 µm.

Furthermore, when the particle of the present invention contains a phospholipid, a water-soluble polymer or the like, adhesion and cohesiveness of the particle itself can be eliminated. As a result, excellent dispersibility and excellent pulmonary inhalation properties are attained and thus it is possible to deliver the compound 1 into the living body at a high ratio through pulmonary administration.

The pulmonary delivery rate of the particle of the present invention is preferably from about 10 to about 100%, and more preferably from about 20 to about 70%.

Criteria for attaining such a pulmonary delivery rate include, for example, an aerodynamic particle size (aerodynamic diameter). The aerodynamic diameter is defined as a particle diameter of a particle which has the same sedimentation rate as the relevant particle and has a density of 1 g/cm³ and is spherical. The aerodynamic particle size can be determined by utilizing a difference in a sedimentation distance of the particles and a difference in inertia when the particles are accelerated. For example, an Andersen cascade impactor used in Examples serves to collect the particles sized according to the aerodynamic particle size. For example, in the case of inhaling at a flow rate of 28.3 L/min, the aerodynamic particle size of the deposited particle to each stage is considered to be 10 µm or more in the throat and a preseparator, 9.0 to 10.0 µm on the stage 0, 5.8 to 9.0 µm on the stage 1, 4.7 to 5.8 µm on the stage 2, 3.3 to 4.7 µm on the stage 3, 2.1 to 3.3 µm on the stage 4, 1.1 to 2.1 µm on the stage 5, 0.65 to 1.1 µm on the stage 6, 0.65 to 0.43 µm on the stage 7, and less than 0.43 µm on the filter. In the present invention, in the case of inhaling at a flow rate of 28.3 L/min, the amount of *in vitro* pulmonary delivery and the rate of *in vitro* pulmonary delivery are calculated assuming that a particle having an aerodynamic particle size of 4.7 µm or less to be deposited to stages 3, 4, 5, 6, 7 and a filter is delivered into the lung. Also in the case of inhaling at a flow rate of 60 L/min, the amount of *in vitro* pulmonary delivery and the rate of *in vitro* pulmonary delivery are calculated assuming that a particle having an aerodynamic particle size of 4.7 µm or less to be delivered to stages 2, 3, 4, 5, 6 and a filter is delivered into the lung.

The aerodynamic particle size of a particle used in the pulmonary preparation is preferably from about 0.01 to about 10 µm, and more preferably from about 0.01 to about 4.7 µm.

The particle having the above particle size of the present invention can be obtained by comminuting the compound 1 produced by a known method. The comminution method includes, but is not limited to, dry comminution, wet comminution (for example, comminution in water and the like), ultrasonic comminution and laser comminution, and is preferably wet comminution, more preferably comminution in water.

Since the compound 1 is poorly soluble in water and has high water repellency, it is difficult to suspend the compound 1 in water as it is, and the compound 1 can be comminuted in water using a water-soluble polymer or a phospholipid as a suspending agent. The compound 1 is preferably comminuted in water in the presence of a phospholipid, a water-soluble polymer or the like, and thus a particle having a desired mean particle size can be obtained.

The comminution in water does not mean direct comminution of a substance under dry condition, but comminution in the presence of a liquid which hardly dissolves the substance or does not dissolve the substance at all. Thus, generally, it is possible to give a smaller particle as compared with the case of dry comminution.

In the comminution in water, water is mainly used as the liquid and an organic solvent may be appropriately combined with water. The organic solvent includes, for example, at least one selected from n-hexane, acetone, ethyl acetate, diethyl ether, chloroform, dichloromethane, ethanol, methanol and the like.

The phospholipid is not specifically limited and, for example, natural phospholipids such as soybean lecithin are preferable, and hydrogenated soybean lecithin obtained by hydrogenating soybean lecithin is also preferable.

As the water-soluble polymer, water-soluble celluloses are preferably used, and hydroxypropylmethyl cellulose, hydroxypropyl cellulose and methyl cellulose are more preferable.

Soybean lecithin, hydrogenated soybean lecithin, hydroxypropyl cellulose and/or hydroxypropylmethyl cellulose exhibit excellent properties in dispersibility, pulmonary delivery and granulation properties as compared with the case of using other natural phospholipids, DPPC (dipalmitoylphosphatidylcholine), DMPG (dimyristoylphosphatidylcholine), egg yolk lecithin or the lile.

The particle of the present invention may further contain a sugar in addition to a phospholipid or a water-soluble polymer. The sugar includes, for example, saccharides (monosaccharides, disaccharides, polysaccharides and the like), sugar alcohols, other polyols and the like. The saccharides are preferably lactose, glucose, fructose, trehalose, sucrose, raffinose and melezitose, while the sugar alcohols are preferably lactitol, maltitol and D-mannitol, and pullulan and starch are also preferable.

In the present invention, the preparation comprising the particle of the present invention is a preparation comprising a particle which contains only the compound 1 having a given particle size, or further contains a water-soluble polymer, a phospholipid or the like.

The amount of the phospholipid or the water-soluble polymer is preferably from about 1 to about 20 parts by mass, and more preferably from about 3 to about 15 parts by mass, based on 100 parts by mass of the compound 1. When the amount of the phospholipid or the water-soluble polymer is within the above range, the compound 1 can be suitably comminuted in water.

The particle has a particle size suitable for administration as a pulmonary preparation. The particle obtained by comminution and further drying is formed into a preparation having excellent dispersibility as a result of elimination of adhesion and cohesiveness.

In the present invention, drying means removal of a liquid such as water used in the comminution in water and particularly includes drying through granulation, freeze-drying and the like.

For example, granulation is preferably any of stirring granulation, extrusion granulation, spray drying granulation, spray drying type fluidized bed granulation, rolling granulation, rolling fluidized bed granulation, pressure swing granulation and centrifugal rolling granulation, and is particularly preferably spray drying granulation.

As a carrier for delivery of the particle of the present invention into the lung in the case of drying the particle of the present invention obtained by comminution in water and/or the preparation base for reduction of adhesion and cohesiveness, pharmacologically acceptable sugar may be used. Adhesion and cohesiveness can be reduced by forming a particle as a comminuted product into a smooth spherical shape.

In the present invention, the sugars may be added as a component for transferring a particle, and also may be added as a carrier for deposition of the particle of the present invention into the lung as described above.

The sugars which can be used in the present invention include saccharides (monosaccharides, disaccharides, polysaccharides and the like), sugar alcohols, other polyols and the like. The saccharides are preferably lactose, glucose, fructose, trehalose, sucrose, raffinose and melezitose, sugar alcohols are preferably lactitol, maltitol and D-mannitol, and pullulan and starch are also preferable. As the lactose, lactose for inhalation is known and also can be used in the present invention. The lactose for inhalation includes, for example, Lactohale LH300, Lactohale LH200, Lactohale LH100 (all of which are trade names, Friesland Foods Domo). As the lactose other than the above, it is also possible to preferably use 450M DMV lactose, 325M DMV lactose, Respitose (all of which are trade names, DMV International Co.), 200M NZ lactose (trade name, Fonterra Co.), Prismalac 40, Capsulac 60, Sachelac 80, Spherolac 100, Inhalac 70, Inhalac 120, Inhalac 230, Granulac 70, Granulac 140, Granulac 200 and Granulac 230 (all of which are trade names, Magle Pharma).

Even if the sugar has a comparatively large particle size, the particle of the present invention can be delivered to the lung at a high ratio. In such a case, the sugar can be used as a carrier, for example. The carrier means mixed particles of a particle (drug particle) containing the compound 1 and a sugar particle in which the drug particle is delivered to a lower respiratory tract such as trachea or bronchus in the case of spraying into the respiratory tract from an inhalator, while the carrier itself stays in an oral cavity, pharynx or larynx and is less likely to deliver to the lung. The mean particle size of the sugar used as the carrier is preferably from about 1 to about 150 µm, and more preferably from about 1 to about 50 µm.

In view of reduction of adhesion and cohesiveness, such sugar particle preferably has a smooth surface, namely, a small surface energy. As the sugar particle having a small surface energy, for example, a spray-dried granule can be preferably used.

In the case of using the particle as a preparation for inhalation, the particle may further contain other preparation bases, for example, surfactants, auxiliary dispersants, excipients, binders, lubricants, disintegrators, auxiliary disintegrators, thickeners, suspending agents, emulsifiers, corrigents, preservatives, stabilizers, pH adjustors, antioxidants, refrigerants and releasants, and at least one of them can be appropriately added and used.

A capsule to be installed in an inhaler (when being used) or a blister may be filled with the preparation for inhalation, or a container such as drug storage tank of the inhaler may be filled with the preparation.

If necessary, the patient can simply take the preparation for inhalation using an inherator by inhaling the container filled with the preparation.

The surfactant includes, for example, sodium dodecylsulfate, oleic acid, diethylene glycol dioleate, tetrahydrofurfuryl oleate, ethyloleate, isopropyl myristate, glyceryl trioleate, glyceryl monolaurate, glyceryl monooleate, glyceryl monostearate, glyceryl monolysinolate, cetyl alcohol, stearyl alcohol, polyethylene glycol 400, cetylpyridinium chloride, sorbitan trioleate (the trade name Span 85), sorbitan monooleate (the trade name Span 80), sorbitan monolaurate (the trade name Span 20), polyoxyethylene hardened castor oil (the trade name HCO-60), polyoxyethylene(20)sorbitan monolaurate (the trade name Tween 20), polyoxyethylene(20)sorbitan monooleate (the trade name Tween 80), lecithin derived from a natural resource (the trade name Epicron), oleyl polyoxyethylene(2) ether (the trade name Bridge 92), stearyl polyoxyethylene(2) ether (the trade name Bridge 72), lauryl polyoxyethylene(4) ether (the trade name Bridge 30), oleyl polyoxyethylene(2) ether (the trade name Genapol 0-020), a block copolymer of oxyethylene and oxypropylene (the trade name Synperonic), stearyl triethanolamine, benzalkonium chloride, benzotonium chloride, glycerin monostearate and polysolvate.

The auxiliary dispersant includes as those other than the above surfactants and the like, for example, hydroxypropyl cellulose, gum arabic, ethanol, a carboxyvinyl polymer, carmellose sodium, an agar powder, citric acid, citric acid sodium, glycerin, magnesium silicate, light silicic acid anhydride, crystalline cellulose, synthetic aluminum silicate, titanium oxide, sucrose fatty acid ester, sodium hydroxide, stearic acid, magnesium stearate, lecithin, D-sorbitol, low substituted hydroxypropyl cellulose, dextrin, cornstarch, sorbitan trioleate, lactose, concentrated glycerin, potatostarch, hydroxyethyl cellulose, hydroxypropylmethyl cellulose, propylene glycol, propylene glycol fatty acid ester, povidone, polyethylene glycol 300, polyethylene glycol 4000, polyethylene glycol 6000, polyoxyethylene nonylphenyl ether, macrogol, isopropyl myristate, methyl cellulose, liquid paraffin and calcium hydrogen phosphate.

The excipient includes, for example, saccharose, lactose, D-mannitol, starch, cornstarch, crystalline cellulose, glucose, mannite, sorbit, maltose and light silicic acid anhydride.

The binder includes, for example, crystalline cellulose, D-mannitol, dextrin, starch, methyl cellulose, hydroxypropyl cellulose, hydroxypropylmethyl cellulose, polyvinyl pyrrolidone, polyethylene glycol, gelatine, carboxymethyl cellulose sodium, saccharose and sucrose.

The lubricant includes, for example, magnesium stearate, calcium stearate, light silicic acid anhydride, talc, sodium dodecylsulfate and colloidal silica.

The disintegrator and the auxiliary disintegrator include, for example, starch, carboxymethyl cellulose, carboxymethyl cellulose calcium, crosscarmellose sodium, sodium carboxymethyl starch and L-hydroxypropyl cellulose.

The thickener includes, for example, glycerin, polyhydric alcohols (macrogol and the like), methyl cellulose, carboxymethyl cellulose, sodium carboxymethyl cellulose, hydroxypropylmethyl cellulose, hydroxymethyl cellulose, hydroxyethyl cellulose, hydroxypropyl cellulose, sodium alginate, chondroitin sulfuric acid, cyclodextrin and a carboxyvinyl polymer.

The suspending agent and the emulsifier include, as those other than the above surfactants and the like, polyhydric alcohols (for example, macrogol and the like), sorbitol, D-mannitol, sucrose, methyl cellulose, carboxymethyl cellulose, sodium carboxymethyl cellulose, hydroxypropylmethyl cellulose, hydroxymethyl cellulose, hydroxyethyl cellulose, hydroxypropyl cellulose, chondroitin sulfate, polyvinyl alcohol, polyvinyl pyrrolidone, a carboxyvinyl polymer and sorbitan trioleate.

The corrigent includes, for example, citric acid, menthol, glycyrrhizin ammonium salt, glycine and an orange powder.

The preservative includes, for example, sodium benzoate, sodium hydrogen sulfite, paraben, methylparaben, ethylparaben, propylparaben, butylparaben, benzalkonium chloride, benzetonium chloride, chlorhexidine gluconate, cetylpyridinium chloride, chlorobutanol, benzyl alcohol, phenethyl alcohol, sodium dehydroacetate, sorbic acid, sodium sorbate, parachloromethoxyphenol and parachlorometacresol.

The stabilizer includes, for example, citric acid, sodium citrate, sodium sulfite, sodium hydrogen sulfite, sodium hydrogen metasulfite, sodium thiosulfate, Rongalite, thioglycerol, thioglycolic acid, thiolactic acid, cysteine, glutathione, thioacetic acid, methionine, thiosorbitol, thioglucose, thiourea, boric acid, borax, phosphoric acid, metaphosphoric acid, sodium carbonate, sodium hydrogen carbonate, formic acid, oxalic acid, tartaric acid, citric acid, edetic acid, sodium edetate, acetamide, diethylacetamide, nicotinic-acid amide, urea, barbital, glycol, propylene glycol, glycerin, polyethylene glycol, glucose, ascorbic acid, phenol, thymol, quinone, cumarone, isocumarone, dibutylhydroxytoluene, glycine, glutamic acid, lysin, phenylalanine, caseine and edestin.

The pH adjustor includes, for example, sodium hydroxide, potassium hydroxide, trisodium phosphate, disodium hydrogen phosphate, hydrochloric acid, sulfuric acid, nitric acid, citric acid, boric acid and acetic acid.

The antioxidant includes, for example, sulfite, ascorbic acid, citric acid and sodium edetate.

The refrigerant includes, for example, 1-menthol, dl-menthol, camphor and peppermint water.

The preparation containing the particle of the present invention can be used in combination with aromatics, desiccants, synergists, preservatives, propellants, fluidizing agents, plasticizers and buffers.

In the case of forming the particle of the present invention into the preparation, it is preferred to control the particle size of the compound 1 to the size suited for inhalation so as to deriver the compound 1 into the lung tissue by inhalation and to improve the transfer of the compound 1 from the absorption site to the blood flow.

Namely, the particle size of the particle obtained by comminution of the compound 1 in water is controlled to the size suited to aerodynamical delivery into the lung tissue by spray drying granulation, freeze-drying or the like.

Commonly, pulmonary administration is often used for treatment of respiratory tract diseases. The particle containing the compound 1 is absorbed into blood through the alveolus region having a wide surface area and the action is quickly exhibited. Therefore, the particle can also be applied to drugs for treatment and/or prevention of systemic diseases.

As for the particle having a mean particle size of about 0.01 to about 20 µm, comprising the compound 1, the content of the compound 1 in the entire particle is preferably from about 60 to about 100 w/w%, more preferably from about 75 to about 95 w/w%, and still more preferably from about 80 to about 95 w/w%.

The particle containing the compound 1 of the present invention can be used as, for example, an aerosol, a pressurized metered dose inhaler and a dry powder for inhalation.

Depending on the patient's spontaneous respiration, pulmonary administration can be carried out for local and whole body therapy using a dispersible preparation for inhalation or aerosol filled into a capsule, blister or a drug storage tank of an inhaler.

As a preferable capsule for use in the present invention, for example, commercially available products (for example, a gelatine capsule, a polyethylene glycol-containing gelatine capsule and a hydroxypropylmethyl cellulose capsule provided by Capsugel Co. and Qualicaps Co.) can be used. Specific examples of the material of the capsule include gelatine, hydroxypropylmethyl cellulose, hydroxyethyl cellulose, hydroxypropyl cellulose, hydroxyethylmethyl cellulose and pullulan.

The preparation for pulmonary administration is prepared by a conventional method. For example, a particle containing the compound 1 as an active ingredient can be mixed with a pharmacologically acceptable additive to form a uniform mixture. Furthermore, a capsule, a blister, or a drug storage tank of an inhaler is filled with the mixture. In the case of the capsule or blister, it is further installed into an inhaler and the particle is usually inhaled from the inhaler through intake of the patient and then deposited in the lung as a drug particle.

Also, the inhaler may be directly filled with the preparation comprising the particle of the present invention, and the patient may inhale the particle.

The "inhaler" used in the present specification is not specifically limited, but is preferably a "dry powder inhaler". The "dry powder inhaler" means an inhalation device (passive dry powder inhaler) in which a drug preparation in the device is dispersed and aerosolized by a patient's spontaneous breathing, and also may comprise a means for providing energy so as to disperse and aerosolize the drug preparation, for example, an inhalation device (active dry powder inhaler) comprising a compressed gas and a vibration or rotation element.

The dry powder inhaler is preferably designed so that a particle is protected from moisture and there is no risk of excessive administration of the particle. Furthermore, it is desired that the compound 1 is stably maintained, the compound is highly deposited on the lung as an absorption site, pool in the mouth of the compound is minimized by minimizing errors due to dose, the compound is scarcely deposited on the dry powder inhaler, and the inhalation resistance is small, and when the dry powder is taken from the dry powder inhaler after it is installed into a capsule and a blister, the particle is scarcely deposited on the capsule and the blister. The powder inhalator includes, but is not limited to, various inhalators such as Monohaler, Jethaler, Spinhaler, Dischaler, E-haler, Easyhaler, Terbuhaler, Rotohaler, Ultrahaler, Handyhaler, Taifun, Acuhaler, Skyhaler, Twisthaler, Bedhaler, Gyrohaler, Novolizer, Blisterinhaler and Directhaler.

The preparation for pulmonary administration in the present invention can also be formed into an aerosol. The aerosol means a preparation which is sprayed mistily, foamily or powderly through pressure (when being used) after a pressurized container equipped with an injector (valve), referred to as a pressurized metered dose inhalator (pMDI), is filled with a solution, an emulsion or a suspension of a drug, together with a propellant (for example, a chlorofluorocarbon alternative, a liquefied gas propellant (for example, fluorohydrocarbon, liquefied petroleum, diethyl ether, dimethyl ether and the like), a compressed gas (for example, a soluble gas (for example, carbon dioxide, a dinitrogen monoxide gas and the like), an insoluble gas (for example, a nitrogen gas and the like) or the like)). Upon administration, when the propellant is released from the pMDI under pressure, the propellant is vaporized and the dissolved and dispersed drug is usually deposited in the respiratory tract in the form of a fine particle powder of the drug.

### Application for drug

The particle of the present invention is useful as a bulk powder of a drug and exhibits high *in vivo* deposition by pulmonary administration, for example. The preparation obtained by the present invention can be safely used as a drug and exerts an excellent N-type calcium channel inhibitory action on a mammal, especially human, and is therefore useful as a preventive and/or a therapeutic agent for pain (for example, neuropathic pain, cancerous pain, intractable pain, post-operative pain or the like) or the like.

### Examples

The present invention will now be described in more detail by way of Preparation Examples, Comparative Examples, Absorption Test Examples and Test Examples, but the present invention is not limited thereto. In the following inhalation test, a cascade impactor is a evaluation apparatus for a particle size of an inhalation preparation described in the United States Pharmacopeia, 28th Edition, and the basic operation was conducted in accordance with the United States Pharmacopeia, 28th Edition. In the case of forecasting absorbance of the compound into the lung, the pulmonary delivery of the inhalation preparation can be evaluated using the cascade impactor in vitro.

For the measurement of the particle size, a hexane solution (20 mM) of Aerosol OT was used as a disperse medium. Particles to be measured were placed in a test tube and 1 mL of the disperse medium was added to obtain a suspension. The suspension was subjected to an ultrasonic treatment for one minute and the particle size was measured using a laser diffraction particle size distribution analyzer (SALD-2100: Shimadzu Coxporation). In the case of measuring the particle size of particles in the suspension, an aqueous 0.1% Polysorbate 80 solution was used as the disperse medium in place of Aerosol OT.

### Preparation Example 1: Production of suspension

HPC (hydroxypropyl cellulose)-SL (150 g) and sodium dodecylsulfate (SDS) (1.5 g) were dissolved in purified water (2,548.5 g) and then a compound 1 (300 g) was suspended. Then, the suspension was comminuted in water using a mill to obtain a suspension for pulmonary administration (3,000 g) (theoretical concentration: 10 w/w%, quantitative concentration: 8.37 w/w%). The mean particle size of the compound 1 in the suspension was 201 nm.

### Preparation Example 2: Production of suspension

HPC-SL (100 g) and sodium dodecylsulfate (1 g) were dissolved in purified water (1,699 g) and the compound 1 (200 g) was suspended. Then, the suspension was comminuted in water using a mill to obtain a suspension (2,000 g) (theoretical concentration: 10 w/w%, quantitative concentration: 9.69 w/w%) of the compound 1. The suspension (5 g) was diluted with a purified water (94.389 w/w%) solution (57.5 g) of HPC-SL (5.556 w/w%) and sodium dodecylsulfate (0.056 w/w%) to obtain a suspension for pulmonary administration. The mean particle size of the compound 1 in the resulting suspension was 395 nm.

### Comparative Example 1: Production of preparation for oral administration

d-α-tocoferil polyethylene glycol 1000 succinic acid (2.5 mL) and polyethylene glycol (2.5 mL) were mixed and the compound 1 (40 mg) was added to the resulting mixed solution and then dissolved therein by heating. Then, purified water (15 mL) was added to the mixed solution to obtain a microemulsion preparation for oral administration (20 mL) (concentration: 2 mg/mL).

### Comparative Example 2: Production of preparation for nasal administration

A suspension was prepared by adding the compound 1 and hydroxypropylmethyl cellulose acetate succinate (AQOAT AS-LF) to a mixed solvent of acetone and ethanol (1/1) so as to control a solid content to 7.5% and to control a mass ratio of the compound 1 to hydroxypropylmethyl cellulose acetate succinate to 1:2. The resulting suspension was subjected to spray drying granulation using TCSD (NIPPON SHARYO, LTD.) to obtain a spray-dried granule. The resulting granule (200 g) and magnesium stearate (2 g) were placed in FM-VG01 (Powlex Corporation) and a 1/15 M phosphate buffer (100 mL) was sprayed over 20 g/min., followed by high-speed stirring granulation, wet sizing using a comil (φ 4.75) and further drying using a fluidized bed granulator. The resulting granule was sized using a roll granulator to obtain a granule having a particle size of 75 to 180 µm. The granule was mixed with Supertub and a hydroxypropylmethyl cellulose capsule (No. 2) was filled with 20 mg of the resulting preparation powder having a drug content of 8%.

### Comparative Example 3: Production of suspension for intravenous administration

To a mixed solution of polyoxyethylene hydroxystearate and propylene glycol of Japanese Pharmacopeia grade (7/3), the compound 1 (20 mg) was added, followed by mixing at about 70 °C. The compound was dissolved at about 60°C by adding 1 M hydrochloric acid (24.79 µL) and then water was added to make 5 mL. To the resulting stock solution, a McIlvaine buffer (pH 4.0, 15 mL) was added, followed by stirring to obtain a solution containing 1 mg/mL of the compound 1. The McIlvaine buffer having a pH of 4.0 was obtained by adding an aqueous 0.05 M disodium hydrogen phosphate solution to an aqueous 0.025 M citric acid solution.

### Absorption Test Examples

### 1. Pulmonary administration (2 mg/kg)

Each of three male rhesus monkeys (mean body weight: 3.35 kg, age: 5) was anesthetized with ketamine and, after inserting a bronchial catheter and inserting an administration tube inside the catheter, the suspension produced in Preparation Example 1 was administered into the lung in a dose of 2 mg/kg. Before and after administration (0.25, 0.5, 1, 2, 3, 4, 6, and 8 hours), blood was collected from the cubital cutaneous vein and the obtained blood was centrifugally separated to obtain blood plasma, and then the concentration of the compound 1 was measured.

### 2. Pulmonary administration (0.2 mg/kg)

Each of three male cynomolgus monkeys (mean body weight: 3.5 to 4.5 kg, age: 5 to 6) was anesthetized with ketamine and, after inserting a bronchial catheter and inserting an administration tube inside the catheter, the suspension produced in Preparation Example 2 was administered into the lung in a dose of 0.2 mg/kg. Before and after administration (0.25, 0.5, 1, 2, 3, 4, 8 and 12 hours), blood was collected from the cephalic vein and the obtained blood was centrifugally separated to obtain blood plasma, and then the concentration of the compound 1 was measured.

### 3. Oral administration (3 mg/kg)

An oral administration tube was inserted through the mouth of each of three male rhesus monkeys (mean body weight: 3.5 kg, age: 3) and 5.25 mL (3 mg/kg) of the preparation produced in Comparative Example 1 was orally administrated. After administration (0.5, 1, 2, 4, and 6 hours), blood was collected from the cubital cutaneous vein and the obtained blood was centrifugally separated to obtain blood plasma, and then the concentration of the compound 1 was measured.

### 4. Nasal administration (0.4 mg/kg)

An administration port of Jetlizer was inserted into the nose of each of three male cynomolgus monkeys (mean body weight: 4 kg, age: 5 to 6) and the preparation produced in Comparative Example 2 was nasally administered in an amount of 1 capsule/one nostril (0.4 mg/kg per both nostrils). After administration (0.25, 0.5, 1, 2, 3, 4, 6, 8 hours), blood was collected from the cubital cutaneous vein and the obtained blood was centrifugally separated to obtain blood plasma, and then the concentration of the compound 1 was measured.

### 5. Intravenous administration (0.4 mg/kg)

The preparation produced in Comparative Example 3 was intravenously administered to each of three male cynomolgus monkeys (mean body weight: 4 kg, age: 5 to 6) in the concentration of 0.4 mg/kg. After administration, blood was collected from the cubital cutaneous vein and the obtained blood was centrifugally separated to obtain blood plasma, and then the concentration of the compound 1 was measured.

### Results

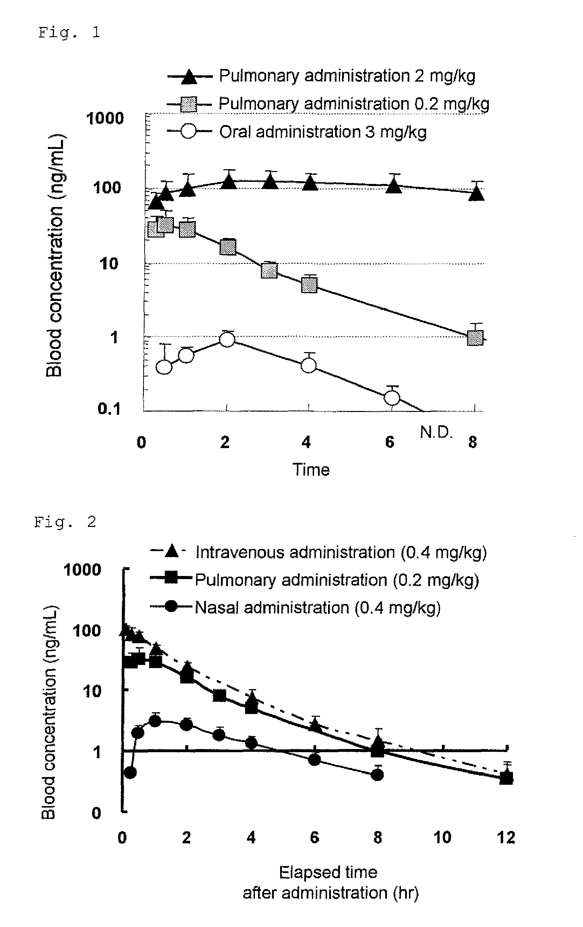

Temporal transitional changes in concentration of the compound 1 in blood plasma in pulmonary administration and oral administration are shown in Fig. 1.

Also, a bioavailability (BA (%)) was calculated from the obtained results.

BA denotes a numerical value represented by "(AUC of pulmonary administration/dose of pulmonary administration)/(AUC of intravenous administration/dose of intravenous administration) x 100" (%), and AUC denotes an area under the blood concentration time curve (ng·hr/ml).

As a result, BA in the case of oral administration was 0.2%, whereas, BAs in the case of pulmonary administration were 103.4% (0.2 mg/kg) and 93.4% (2 mg/kg), namely, nearly 100% in both cases, and thus an improvement in BA by 450 times or more was recognized. As is apparent from these results, absorbency is remarkably improved by the preparation for pulmonary administration containing the compound 1.

The temporal transitional changes in concentration of the compound 1 in blood plasma in intravenous administration, pulmonary administration and nasal administration are shown in Fig. 2. As is apparent from the results, absorbency is remarkably improved by the preparation for pulmonary administration containing the compound 1 as compared with the nasal administration.

### Preparation Example 3: Mixing of compound 1 with lactose

Using a fine impact mill 100UPZ (Hosokawa Micron Corporation), the compound 1 (1.2 kg) was dry-comminuted by a pin-disc (turnover number: 17,500 rpm) to obtain a comminuted product having a mean particle size of 4.5 µm. The comminuted product (3 g) was mixed with each of three kinds of lactose (trade names: Lactohale LH300 (50% diameter: 3 µm), Lactohale LH200 (50% diameter: 90 µm) and Lactohale LH100 (50% diameter: 120 µm), all of which are manufactured by Friesland Foods Domo) (3 g). Using a sieve having a pore size of 355 µm, sieving and mixing were carried out three times to obtain mixtures with three kinds of formulations shown in the following Table 1 (the numerical values mean relative masses).

**Table 1**

| | Formulation 3-1 | Formulation 3-2 | Formulation 3-3 |
|---|---|---|---|
| Dry comminuted product of compound 1 | 10 | 10 | 10 |
| Lactohale LH300 | 10 | - | - |
| Lactohale LH200 | - | 10 | - |
| Lactohale LH100 | - | - | 10 |

### Preparation Example 4: Production of preparation comprising compound 1 and HPMC through comminution in water and granulation

Using a fine impact mill 100UPZ (Hosokawa Micron Corporation), the compound 1 (1.2 kg) was comminuted by a pin-disc (turnover number: 17,500 rpm). The comminuted product (20 g) was suspended in an aqueous 0.5% hydroxypropylmethyl cellulose (TC5E, Shin-Etsu Chemical Co., Ltd.) solution (380 g) and then comminuted in water using a microfluidizer M-110-E/H (MIZUHO Industrial CO., LTD.) under a pressure of 150 MPa to obtain a suspension of the compound 1 having a mean particle size of about 0.95 µm. This suspension (50 g) was spray-dried using a minispray dryer B-290 (heat input temperature: 200°C, heat output temperature: 85 to 90°C, aspirator flow rate: 27 m³/hour, liquid feed speed: 2 to 2.5 g/min., spray air flow rate: 601 L/hour) to obtain a granule (1.8 g). The resulting granule was sieved through a sieve having a pore size of 355 µm to obtain a preparation with the formulation shown in Table 2 (the numerical values mean relative masses).

**Table 2**

| Formulation | Formulation 4 |
|---|---|
| Compound 1 comminuted in water (mg) | 10 |
| HPMC (mg) | 1 |

### Preparation Example 5: Production of preparation comprising compound 1 and hydrogenated soybean lecithin through comminution in water and granulation

Using a fine impact mill 100UPZ (Hosokawa Micron Corporation), the compound 1 (1.2 kg) was dry-comminuted by a pin-disc (turnover number: 17,500 rpm) and the comminuted product (60 g) was suspended in an aqueous hydrogenated soybean lecithin (1.5%H-purified soybean lecithin, AJINOMOTO HEALTHY SUPPLY, INC.) solution (340 g) and then comminuted in water using a microfluidizer M-110-E/H (MIZUHO Industrial CO., LTD.) under a pressure of 150 MPa to obtain a suspension of the compound 1 having a mean particle size of about 0.6 µm. To this suspension (15 g), purified water (30 g) was added and the mixture was subjected to spray drying granulation using a minispray dryer B-290 (heat input temperature: 200°C, heat output temperature: 85 to 90°C, aspirator flow rate: 27 m³/hour, liquid feed speed: 2 to 2.5 g/min., spray air flow rate: 601 L/hour) to obtain a granule (2.1 g). The resulting glanule was sieved through a sieve having a pore size of 355 µm to obtain a preparation with the formulation shown in Table 3 (the numerical values mean relative masses).

**Table 3**

| Formulation | Formulation 5 |
|---|---|
| Compound 1 comminuted in water (mg) | 10 |
| Hydrogenated soybean lecithin (mg) | 1 |

In the case of respectively using citric acid and DK ester (sucrose fatty acid ester) in place of the hydrogenated soybean lecithin, a suspension was able to be prepared, but a fine particle was not able to be obtained by comminution in water. In the case of respectively using dipalmitoylphosphatidylcholine and dimyristoylphosphatidyl glycerol, the suspension was able to be prepared, but the particle was not sufficiently dispersed. In the case of respectively using creatinine, chlorobutanol, nicotinic-acid amide, macrogol 4000, carmellose sodium and sodium hyaluronate, the compound 1 was not able to be sufficiently mixed with water.

### Preparation Example 6: Production of preparation comprising compound 1, hydrogenated soybean lecithin and hydroxypropylmethyl cellulose through comminution in water and granulation

Using a fine impact mill 100UPZ (Hosokawa Micron Corporation), the compound 1 (1.2 kg) was comminuted by a pin-disc (turnover number: 17,500 rpm). The comminuted product (20 g) was suspended in an aqueous 0.5% hydroxypropylmethyl cellulose (TC5E ; Shin-Etsu Chemical Co., Ltd.) solution (380 g) and then comminuted in water under a pressure of 150 MPa using a microfluidizer M-110-E/H (MIZUHO Industrial CO., LTD.) to obtain a suspension of the compound 1 having a mean particle size of about 0.95 µm. To this suspension (50 g), hydrogenated soybean lecithin (1.5%H-purified soybean lecithin: 2.5 mg) was added and, after mixing for one hour, the mixture was subjected to spray drying granulation using a minispray dryer B-290 (heat input temperature: 200°C and heat output temperature: 85 to 90°C, aspirator flow rate: 27 m³/hour, liquid feed speed: 2 to 2.5 g/min. and spray air flow rate: 601 L/hour) to obtain a granule (1.9 g). The resulting glanule was sieved through a sieve having a pore size of 355 µm to obtain a preparation with the formulation shown in Table 4 (the numerical values mean relative masses).

**Table 4**

| Formulation | Formulation 6-1 | Formulation 6-2 | Formulation 6-3 |
|---|---|---|---|
| Compound 1 (mg) | 10 | 10 | 10 |
| HPMC (mg) | 1 | 1 | 1 |
| Hydrogenated soybean lecithin (mg) | 0.01 | 0.05 | 0.1 |

### Preparation Example 7: Production of dry comminuted particle of compound 1

Using a fine impact mill 100UPZ (Hosokawa Micron Corporation), the compound 1 (1.2 kg) was dry-comminuted by a pin-disc (turnover number: 17,500 rpm) to obtain a preparation with Formulation 7. The compound 1 had a mean particle size of about 4.5 µm.

### Preparation Example 8: Preparation obtained by adding lactose to particle comminuted in water and spray drying

Using a fine impact mill 100UPZ (Hosokawa Micron Corporation), the compound 1 (1.2 kg) was comminuted using a pin-disc (turnover number: 17,500 rpm). The comminuted product (20 g) was suspended in an aqueous 0.5% hydroxypropylmethyl cellulose (TC5E, Shin-Etsu Chemical Co., Ltd.) solution (380 g) and then comminuted in water under a pressure of 150 MPa using a microfluidizer M-110-E/H (MIZUHO Industrial CO., LTD.) to obtain a suspension of the compound 1 having a mean particle size of about 0.95 µm. To this suspension (50 g), NZ lactose (0.05 g, 0.25g, 0.5 g or 1 g: manufactured by Fonterra Co-operative Group Ltd.) was added and the mixture was spray-dried using a minispray dryer B-290 (heat input temperature: 200°C, heat output temperature: 85 to 90°C, aspirator flow rate: 27 m3/hourr liquid feed speed: 2 to 2.5 g/min., spray air flow rate: 601 L/ hour) to obtain a granule (1.8 8 g). The resulting granule was sieved through a sieve having a pore size of 355 µm to obtain a preparation with the formulation shown in Table 5 (the numerical values mean relative masses).

**Table 5**

| | Formulation 8-1 | Formulation 8-2 | Formulation 8-3 | Formulation 8-4 |
|---|---|---|---|---|
| Compound 1 (mg) | 10 | 10 | 10 | 10 |
| HPMC (mg) | 1 | 1 | 1 | 1 |
| NZ lactose | 0.2 | 1 | 2 | 4 |

### Test Example 1: Measurement of rate of in vitro pulmonary delivery using inhaler

Using the preparations with Formulation 3-1, Formulation 3-2 and Formulation 3-3 prepared in Preparation Example 3, each rate of *in vitro* pulmonary delivery was determined using a cascade impactor. A hydroxypropylmethyl cellulose (HPMC) capsule (No. 2) was filled with a sample so that the content of the compound 1 is 20 mg. The capsule was placed in Jethaler (reverse chamber: Hitachi, Ltd.) as a dry powder inhaler, followed by inhalation using a cascade impactor at an inhalation flow rate of 28.3 L/min for 8 seconds.

The residual amount of the compound 1 and the amount of delivered compound 1 in 12 fractions of a capsule, an induction port, a preseparator, stage 0, stage 1, stage 2, stage 3, stage 4, stage 5, stage 6, stage 7 and a filter were determined, and also the residual amount in the inhaler was calculated by a change in mass. As the amount of *in vitro* pulmonary delivery, using 6 fractions of 4.7 µm stage 3, stage 4, stage 5, stage 6, stage 7 and a filter as the content delivered to the lung, the total amount of the compound 1 and the rate of *in vitro* pulmonary delivery (proportion of the amount of *in vitro* pulmonary delivery based on the preparation) was measured. The results are shown in Table 6, together with the preparation with Formulation 7 obtained without adding lactose.

**Table 6**

| Formulation | Formulation 3-1 | Formulation 3-2 | Formulation 3-3 | Formulation 7 |
|---|---|---|---|---|
| Mean particle size of comminuted product (µm) | 4.5 | 4.5 | 4.5 | 4.5 |
| *In vitro* pulmonary delivery (%) | 19 | 16.1 | 8.4 | 9.0 |
| Amount of *in vitro* pulmonary delivery (mg) | 3.8 | 3.2 | 1.7 | 1.8 |

As a result, the preparations obtained by using Lactohale LH300 (Formulation 3-1) and Lactohale LH200 (Formulation 3-2), each having a small particle size, showed better pulmonary delivery as compared with the preparation obtained by using Formulation 7 in which lactose is not added and the preparation obtained by using Lactohale LH100 (Formulation 3-3) having a large particle size.

### Test Example 2: Evaluation of rate of in vitro pulmonary delivery using inhaler

### (1) In the case of using Jethaler as inhaler

Using Jethaler as an inhaler, pulmonary delivery of the preparation with Formulation 6-1 of Preparation Example 6 was measured by the same operation as in the method described in Test Example 1.

### (2) In the case of using Monohaler as inhaler

A hydroxypropylmethyl cellulose capsule (No. 3) was filled with the preparation with Formulation 6-1 of Preparation Example 6 so that the content of the compound 1 is 20 mg. The capsule was placed in Monohaler (Miat Co.), followed by inhalation using a cascade impactor at an inhalation flow rate of 60 L/min for 4 seconds. The residual amount of the compound 1 and the amount of delivered compound 1 in 12 fractions of a capsule, an induction port, a preseparator, stage -1, stage 0, stage 1, stage 2, stage 3, stage 4, stage 5, stage 6 and a filter were determined, and also the residual amount in the inhaler was calculated by a change in mass. As the amount of *in vitro* pulmonary delivery, *in vitro* delivery of the compound 1 delivered to 6 fractions of stage 2, stage 3, stage 4, stage 5, stage 6 and a filter was measured. The results are shown in Table 7.

**Table 7**

| Inhalation method | Inhalation method 1 (Jethaler) | Inhalation method 2 (Monohaler) |
|---|---|---|
| Mean particle size of comminuted product (µm) | 0.95 | 0.95 |
| *In vitro* pulmonary delivery (%) | 45 | 52 |
| Amount of *in vitro* pulmonary delivery (mg) | 39 | 51 |

As a result of the evaluation of *in vitro* inhalation characteristics using a cascade impactor, it was suggested that a particle containing about 45 to 52% of the compound 1 can be delivered into the lung as a therapeutic range.

As shown in Table 7, although inhalation characteristics are preferable in the case of using Jethaler and Monohaler, inhalation characteristics are more preferable in the case of using Monohaler as an inhaler. Monohaler is preferable because it has a more simple structure as compared with Jethaler and is less likely to leave the particle containing the compound 1 in the inhaler, and also causes small pressure loss and is easy to perform inhalation.

As a result, it was shown that, according to the present invention, it is possible to prepare a particle which can be efficiently delivered into the lung and to design a particle of the compound 1 which is useful for treatment of intractable pain.

### Test Example 3: Lung deposition rate of preparation containing hydroxypropylmethyl cellulose or hydrogenated soybean lecithin

Using Jethaler as an inhaler, each delivery of the preparation with Formulation 4 produced in Preparation Example 4 and the preparation with Formulation 5 produced in Preparation Example 5 was studied by the same operation as in the method described in Test Example 1. The results are shown in Table 8.

**Table 8**

| Formulation | Formulation 4 | Formulation 5 |
|---|---|---|
| Mean particle size of comminuted product (µm) | 0.95 | 0.6 |
| *In vitro* pulmonary delivery (%) | 36 | 32 |
| Amount of *in vitro* pulmonary delivery (mg) | 7.0 | 6.3 |

As shown in Table 8, by mixing the compound 1 having a mean particle size of 0.6 or 0.95 µm with hydroxypropylmethyl cellulose or hydrogenated soybean lecithin, the compound 1 is delivered into the lung in a high proportion.

### Test Example 4: Pulmonary delivery rate of preparation containing hydroxypropylmethyl cellulose and hydrogenated soybean lecithin

Using Jethaler as an inhaler, each delivery of the preparations with Formulation 6-1, Formulation 6-2 and Formulation 6-3 produced in Preparation Example 6 was measured by the same operation as in the method described in Test Example 1. The results are shown in Table 9.

**Table 9**

| Formulation | Formulation 6-1 | Formulation 6-2 | Formulation 6-3 |
|---|---|---|---|
| Mean particle size of comminuted product (µm) | 0.95 | 0.95 | 0.95 |
| *In vitro* pulmonay delivery(%) | 37 | 38 | 37 |
| Amount of *in vitro* pulmonay delivery (mg) | 8.2 | 8.7 | 8.2 |

As shown in Table 9, the preparation with Formulation 4, which contains the compound 1 having a mean particle size of 0.95 µm and does not contain hydrogenated soybean lecithin, and the preparations with Formulations 6-1, 6-2 and 6-3, which contain hydrogenated soybean lecithin, show high pulmonary delivery.

### Test Example 5: Study of delivery of dry comminuted preparation composed only of compound 1 into lung using inhaler

Using Monohaler as an inhaler, each delivery of the preparation with Formulation 7 produced in Preparation Example 7 and the original drug of the compound 1 into the lung was measured by the same operation as in the method described in Test Example 2(2). The results are shown in Table 10.

**Table 10**

| | Formulation 7 | Original drug of compound 1 |
|---|---|---|
| Mean particle size (µm) | 4.5 | 120.0 |
| *In vitro* pulmonary delivery (%) | 26.9 | 1.1 |

As shown in Table 10, the particle having a mean particle size of 4.5 µm shows better *in vitro* pulmonary delivery rate as compared with the original drug having a mean particle size of 120 µm.

### Test Example 6: Pulmonary delivery rate in the case of mixing particle of compound 1 with various lactoses

Using the particle (particle size: 4.5 µm) of the compound 1 prepared in Preparation Example 7 and various lactoses, the following preparations were obtained by the same operation as in the method described in Preparation Example 3. Using Jethaler as an inhaler, delivery of each mixture into the lung was studied by the same operation as in the method descried in Test Example 1. The results are shown in Table 11.

**Table 11**

| Kind of lactose | 450M DMV lactose | 325M DMV lactose | 200M NZ lactose |
|---|---|---|---|
| Mean particle size of comminuted product (µm) | 4.5 | 4.5 | 4.5 |
| *In vitro* pulmonary delivery (%) | 15 | 16.1 | 12.8 |
| Amount of *in vitro* pulmonary delivery (mg) | 3 | 3.2 | 2.6 |

As shown in Table 11, even when using lactose other than the lactose used in Test Example 1, the particle of the compound 1 obtained by dry comminution shows a high pulmonary delivery rate by mixing 450M DMV lactose, 325M DMV lactose or 200M NZ lactose.

### Test Example 7: Measurement of delivery of inhaled particle obtained using comminution in water in combination with spray drying granulation into lung

Using Jethaler as an inhaler, each delivery of the preparation with Formulation 7 produced in Preparation Example 7, the preparation with Formulation 4 produced in Preparation Example 4 and the preparations with Formulations 8-1, 8-2 and 8-3 produced in Preparation Example 8 into the lung were measured by the same operation as in the method described in Test Example 1. The results are shown in Table 12.

**Table 12**

| | Formulation 7 | Formulation 4 | Formulation 8-1 | Formulation 8-2 | Formulation 8-3 | Formulation 8-4 |
|---|---|---|---|---|---|---|
| Particle size of comminuted product (µm) | 4.5 | 0.95 | 0.95 | 0.95 | 0.95 | 0.95 |
| Compound 1 (mg) | 10 | 10 | 10 | 10 | 10 | 10 |
| HPMC (TC5) | - | 1 | 1 | 1 | 1 | 1 |
| NZ lactose | - | - | 0.2 | 1 | 2 | 4 |
| *In vitro* pulmonary delivery (%) | 9.0 | 34.9 | 36.0 | 35.7 | 32.3 | 33.6 |
| Amount of *in vitro* pulmonary delivery (mg) | 1.8 | 7.0 | 7.2 | 7.1 | 6.5 | 6.7 |

As shown in Table 12, any preparation having a mean particle size of 0.95 µm showed higher pulmonary delivery rate as compared with the case of administering the comminuted product having a mean particle size of 4.5 µm.

Although each preparation shows good pulmonary delivery regardless of the presence or absence of lactose, dispersibility of particles is improved by mixing lactose, and thus it becomes easy to handle the preparation.

As shown in the above Test Examples, the preparation containing the particle of the present invention is excellent in a pulmonary delivery rate through inhalation. Also, as shown in the results of Absorption Test Examples, the compound 1 delivered into the lung tissue exhibits high blood concentration, and thus the particle of the present invention is useful as a therapeutic agent for diseases in which N-type calcium channel of the compound 1 is present.

### Industrial Applicability

The particle of the compound 1 is excellent in dispersibility and therefore can be used as a drug which is a pulmonary inhalation preparation. The compound 1 has an N-type calcium channel inhibitory action and is therefore useful for treatment and/or prevention of neurogenic pain and the like.

### Brief Description of the Drawings

Fig. 1 is a graph showing temporal transitional changes in concentration of the compound 1 in blood plasma in pulmonary administration and oral administration.
Fig. 2 is a graph showing temporal transitional changes in concentration of the compound 1 in blood plasma in intravenous administration, pulmonary administration and nasal administration.

## Claims

1. A Particle having a mean particle size of 0.01 to 20 µm, comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate.

2. The particle according to claim 1, which has a mean particle size of 0.03 to 5 µm.

3. The particle according to claim 1, comprising at least one kind selected from a water-soluble polymer and a phospholipid.

4. The particle according to claim 3, wherein the water-soluble polymer is at least one kind selected from hydroxypropylmethyl cellulose, hydroxypropyl cellulose and methyl cellulose and the phospholipid is at least one kind selected from soybean lecithin and hydrogenated soybean lecithin.

5. The particle according to claim 3, which is produced by comminution in water.

6. The particle according to claim 1, further comprising a sugar.

7. The particle according to claim 6, wherein the sugar are lactose, glucose or D-mannitol.

8. A method for producing a particle having a mean particle size of 0.03 to 5 µm, comprising tert-butyl (4R)-4-{[((1R)-2-C(1-benzylpiperidin-4-yl)amino]-1-{[(cyclohexylmethyl)thio]methyl}-2-oxoethyl)amino]carbonyl}-1,3-thiazolidine-3-carboxylate, comprising the step of comminuting a particle of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in water in the presence of at least one kind selected from a water-soluble polymer and a phospholipid.

9. A preparation comprising the particle according to any one of claims 1 to 7.

10. The preparation according to claim 9, which is a preparation for pulmonary administration.

11. The preparation according to claim 9, which is produced by comminution in water and further granulation or freeze-drying.

12. A method for producing a preparation comprising a particle having a mean particle size of 0.03 to 5 µm and comprising tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate, which comprises the steps of comminuting a particle of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in the presence of at least one selected from a water-soluble polymer and a phospholipid, and granulating the comminuted particle through spray drying.

13. A container for inhalation, comprising the preparation according to claim 10.

14. The particle according to claim 1, wherein the content of tert-butyl (4R)-4-{[((1R)-2-[(1-benzylpiperidin-4-yl)amino]-1-{[(cyclohexylmethyl)thio]methyl}-2-oxoethyl)amino]carbonyl}-1,3-thiazolidine-3-carboxylate in the entire particle is from 60 to 100 w/w%.
